# EUROPÄISCHE PATENTSCHRIFT

(11) **EP 0 780 687 B1**
(45) Veröffentlichungstag und Bekanntmachung des Hinweises auf die Patenterteilung: **13.10.2004**
(21) Anmeldenummer: 96119774.6
(22) Anmeldetag: 10.12.1996
(51) Int. Cl.: G01N 33/68, G01N 33/543, C07K 14/47, G01N 33/96

(54) **Verwendung einer synthetischen Kalibrator für Sandwich-Immunoassays**
Use of a synthetic calibrator for sandwich immunoassays
L' utilisation d' un étalon synthétique pour des tests immunologiques sandwich

(30) Priorität: 21.12.1995 DE 19548028
(43) Veröffentlichungstag der Anmeldung: 25.06.1997
(73) Patentinhaber: Bayer HealthCare AG, 51368 Leverkusen (DE)
(72) Erfinder: Doth, Margit, Dr., 47800 Krefeld (DE); Petry, Christoph, Dr., 47800 Krefeld (DE); Petesch, Nicole, Dr, 42115 Wuppertal (DE)

(56) Entgegenhaltungen:
- EP-A- 0 650 053
- WO-A-94/27156
- GB-A- 2 275 774
- WILLIAMS G: "NOVEL ANTIBODY REAGENTS: PRODUCTION AND POTENTIAL" TRENDS IN BIOTECHNOLOGY, Bd. 6, Nr. 2, Februar 1988, Seiten 36-39, 42, XP000009941

## Beschreibung

Zum Nachweis von Proteinen in Serum- oder Urinproben für medizinisch-diagnostische Zwecke werden häufig wegen besonders guter Spezifität und Sensitivität Immunoassays eingesetzt. Dazu bedarf es neben einem oder zwei spezifischen Antikörpern eines Kalibrators, der als Vergleichsmaßstab zur Quantifizierung der Patientenproben benutzt wird. Besonders für automatisierte Assays in großen Analyselaboratorien ist die Lagerung der Kalibratoren bei 4°C für mehrere Wochen bis Monate wünschenswert. Diese Anforderungen an die Stabilität der Kalibratorformulierung können je nach Analyt Schwierigkeiten bereiten, wenn z.B. die Löslichkeit unter physiologischen Salz- und pH-Bedingungen nicht gewährleistet ist. Als Beispiel seien hier Troponin I und Troponin T genannt, die nur in denaturierenden Lösungen (6 M Harnstoff, 0.01 M Dithiothreitol) hinreichend stabil und löslich sind. Mit dieser denaturierenden Formulierung kann jedoch kein Immunoassay etabliert werden, da die Antikörper unter dieser Behandlung leiden.

Es ist bekannt, daß Proteine in Lösung wenig stabil sind und daß sie enthaltende Reagenzien häufig in gefriergetrockneter Form vertrieben werden, mit einem Lösungmittel geeigneter Zusammensetzung, in dem sie vor der Verwendung von dem Benutzer aufgelöst werden müssen. Wenn man die auf diese Weise erhaltenen Lösungen bei 4°C aufbewahrt, kann man sie während mehrerer Tage benutzen, selbst wenn die tägliche Bestimmung eine gewisse Veränderung der Konzentration des Reagens zeigt. So werden im allgemeinen - und es wird auch im Fall von Troponin I und Troponin T empfohlen - die ausgehend von dem gefriergetrockneten Material erhaltenen Vergleichslösungen für längere Lagerung in Einheitsdosisform einzufrieren.

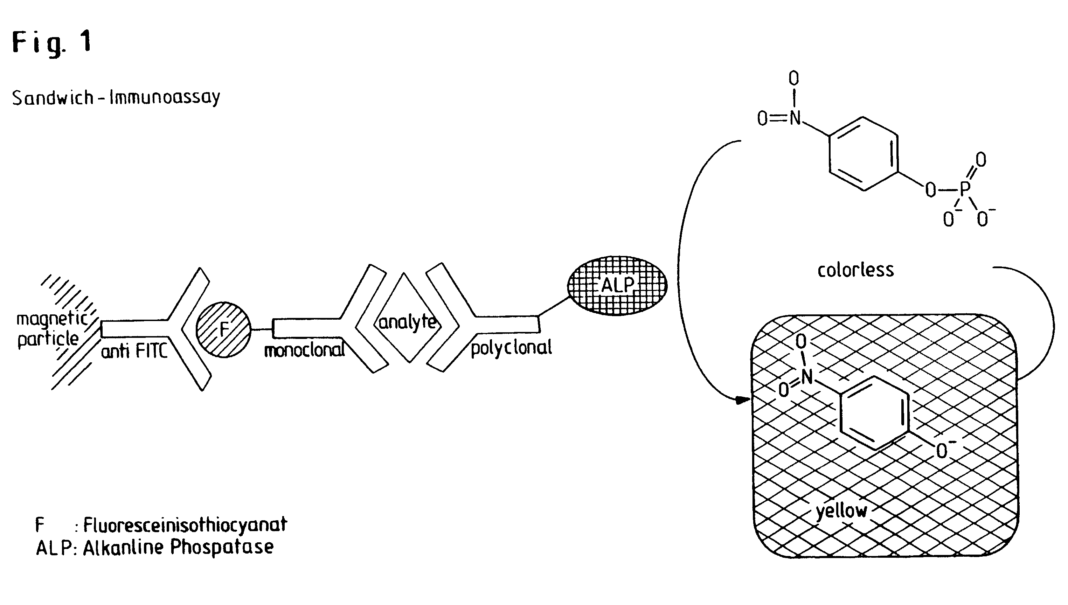

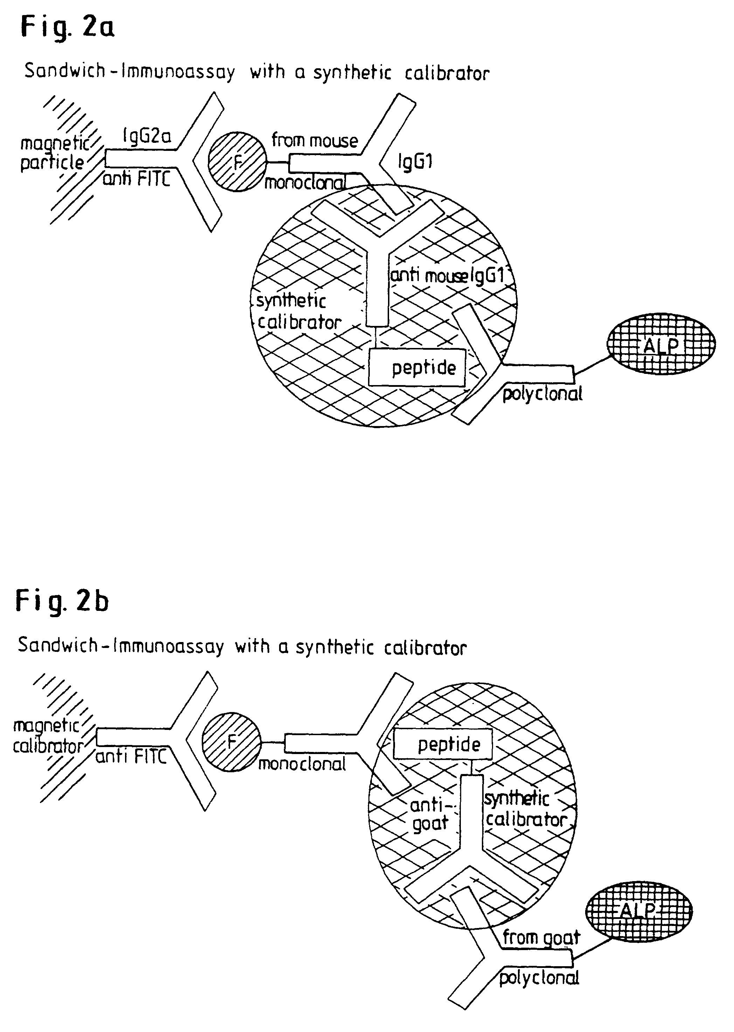

Die vorliegende Erfindung betrifft einen synthetischen Kalibrator mit sehr guter Stabilität für den Einsatz in Sandwich-Immunoassays zur Bestimmung medizinisch relevanter Analyten in Blut-, Plasma-, Serum- oder Urinproben. Die Besonderheit von Sandwich-Immunoassays besteht in der Benutzung zweier für den Analyten spezifischer Antikörper, die an unterschiedlichen Erkennungsstellen (Epitopen) binden, so daß der Analyt zwischen diesen beiden Antikörpern liegt (siehe Abb. 1). Ein Kalibrator für einen Sandwich-Immunoassay muß demnach auch zwei Bindestellen besitzen: eine für jeweils einen der benutzten Antikörper Oft ist das Epitop wenigstens eines Antikörpers in Form der Aminosäuresequenz bekannt, so daß eine Bindestelle aus diesem Peptid, d.h. einer Teilsequenz des Analyten bestehen kann. Ist das Epitop des zweiten Antikörpers unbekannt, oder hat es eine dreidimensionale Struktur, kann diese Erkennungsstelle nicht mit einem Peptid simuliert werden. Um trotzdem einen Sandwich auszubilden, kann man die Bindungsfähigkeit des Antikörpers an einer anderen als der Antigenerkennungsstelle nutzen. Eine Möglichkeit besteht in der Anwendung von Antikörpern, oder Antikörperfragmenten, die gegen den genannten Antikörper gerichtet sind. Konjugiert man an diesen Antikörper an das oben erwähnte Peptid, so erhält man einen synthetischen Kalibrator, der jeweils eine Bindestelle für die im Sandwich-Immunoassay verwendeten Antikörper trägt (siehe Abb. 2a und b).

Die chemische Konjugation verläuft nach bekannten Methoden, die in der Literatur (S.S. Wong, Chemistry of protein conjugation and cross-linking, 1991, CRC Press Inc. ISBN 0-8493-5886-8) beschrieben sind.

Die Erfindung betrifft insbesondere ein synthetisches Kalibratormaterial für das kardiale Troponin I, ein herzspezifisches Protein, das bei der Diagnostik des akuten Myokardinfarktes eine Rolle spielt. Der Kalibrator besteht aus je einem Peptid dieses Analyten, das an Antikörper konjugiert wurde. Diese Antikörper reagieren mit den für den Nachweis des Analyten im Test eingesetzten Antikörpern. Bei den Peptiden handelt es sich um Epitope der analytspezifischen Antikörper, das sind i.d.R. Proteinsequenzen aus der Moleküloberfläche. Sie können mit handelsüblichen Synthesizern hergestellt werden. Unter Peptiden sind auch Peptid-Derivate zu verstehen, in denen eine oder mehrere Aminosäuren durch eine chemische Reaktion derivatisiert worden sind. Beispiele von erfindungsgemäßen Peptidderivaten sind insbesondere solche Moleküle, in denen das Backbone oder/und reaktive Aminosäureseitengruppen, zum Beispiel freie Aminogruppen, freie Carboxylgruppen oder/und freie Hydroxylgruppen, derivatisiert worden sind. Spezifische Beispiele für Derivate von Aminogruppen sind Sulfonsäure- oder Carbonsäureamide, Thiourethanderivate und Ammoniumsalze, zum Beispiel Hydrochloride. Beispiele für Carboxylgruppenderivate sind Salze, Ester und Amide. Beispiele für Hydroxylgruppenderivate sind O-Acyl- oder O-Alkylderivate.

Weiterhin umfaßt der Begriff Peptidderivat auch solche Peptide, in denen eine oder mehrere Aminosäuren durch natürlich vorkommende oder nicht-natürlich vorkommende Aminosäurehomologe der 20 Standard"-Aminosäuren ersetzt werden. Beispiele für solche Homologe sind 4-Hydroxyprolin, 5-Hydroxylysin, 3-Methylhistidin, Homoserin, Ornithin, β-Alanin und 4-Aminobuttersäure. Die Peptidderivate müssen eine im wesentlichen äquivalente Spezifität oder/und Affinität der Bindung an die Antikörper aufweisen, wie die Peptide, aus denen sie abgeleitet sind. Die Länge der Peptide beträgt üblicherweise mindestens 4 Aminosäuren. Bevorzugt liegt die Länge zwischen 4 bis 30 und besonders bevorzugt zwischen 4 bis 15 Aminosäuren.

An den Peptiden wurde C-terminal ein Cystein zur Erleichterung der Konjugation angehängt. Zur Kopplung wurde eine für die Proteinkonjugation bekannte Methode gewählt (S. Yoshitake et al., Eur. J. Biochem., 101:395, 1979). Die Antikörper wurden mit SMCC aktiviert, indem 20 mM SMCC in DMF gelöst und als 25-facher Überschuß zum Antikörper gegeben wurde. Die Mischung inkubierte 25 min bei 25°C. Beendet wurde die Reaktion durch Zugabe von 1uM Glycinlösung (10 min, 25°C). Die Peptide wurden entweder über die Sulfhydrylgruppe in ihrer Sequenz oder durch Einfügen einer solchen mit 2-IT an den aktivierten Antikörper gebunden. Die Anzahl der Peptide pro Antikörper liegt üblicherweise bei 1 bis 50, bevorzugt bei 1 bis 10 und die Anzahl der verschiedene Peptidsequenzen liegt zwischen 1 und 20, bevorzugt 1-5, besonders bevorzugt 1.

Zur Reinigung der Kalibratorsubstanz, d.h. Abtrennung der niedermolekularen Peptide wurde eine Gelchromatographie (Superdex 200) durchgeführt. Anschließend erfolgte UV-spektrometrischen Konzentrationsbestimmung und Stabilisierung über 0.5% BSA/0.1 % Natriumazid. Möglich ist weiterhin eine affinitätschromatographische Reinigung zur Abtrennung unmarkierten Antikörpers über die sequenzspezifischen Antikörper, die auch im Immunoassay eingesetzt werden.

### Beispiele

### Beispiel 1

Ein Peptid mit der Sequenz RAYATEPHAKKKS (Sequenz 1) wurde an einen anti-Maus-Antikörper konjugiert werden. Der Immunoassay lief nach dem Schema in Abb. 2a ab. Der monoklonale Antikörper hatte den Isotyp IgG1. Folglich muß der anti-Maus Kalibratorantikörper gegen IgG1 gerichtet sein. Er reagiert nicht mit IgG2a (anti-FITC) auf den Magnetpartikeln. Der polyklonale Antikörper erkennt das Peptid der genannten Sequenz. Damit wird ein Sandwich gebildelt, bei dem der Analyt TnI durch den synthetischen Kalibrator ersetzt wird.

### Automatisierter Sandwich-Assay

Der künstliche Kalibrator wurde auf dem automatisierten Technicon Analyser Immuno 1® (Bayer Diagnostics) eingesetzt. Das Assay-Format war ein Sandwich unter Benutzung folgender Antikörper: 1. monoklonaler Antikörper gegen humanes kardiales Troponin I, 2. polyklonaler Antikörper aus Ziege, affinitätsgereinigt gegen das Peptid der Sequenz 1. Der erste Antikörper des Sandwich bindet anti-Maus IgG1 des künstlichen Kalibrators. Er ist mit FITC markiert und wird über anti-FITC auf Magnetpartikeln immobilisiert. Der 2. Antikörper des Sandwich reagiert mit dem Peptid auf dem synthetischen Kalibrator. Er trägt alkalische Phosphatase und katalysiert die Farbreaktion. Die Antikörper-Inkubation erfolgte sequentiell. Bei diesem Testverfahren konnte eine der Konzentration der Kalibratorsubstanz proportionale Zunahme der Farbintensität gezeigt werden.

### Beispiel 2

Ein anderer Kalibrator wurde gebildet aus der Sequenz TGLGFAELQDLCRQIHARVD (Sequenz 2) und einem anti-Ziege Antikörper (Abb. 2b). Das Peptid wird dabei von dem monoklonalen Antikörper erkannt. Der anti-Ziege Antikörper des Kalibrators bindet an den polyklonalen Antikörper aus der Ziege, der das Enzym für die Farbreaktion trägt.

### Automatisierter Sandwich-Assay

Der künstliche Kalibrator nach Beispiel 2 wurde ebenfalls auf dem automatisierten Technicon Analyser Immuno 1® (Bayer Diagnostics) eingesetzt. Das Assay-Format war ein Sandwich unter Benutzung folgender Antikörper: 1. monoklonaler Antikörper gegen die Sequenz 2 des humanen kardialen Troponin I, 2. polyklonaler Antikörper aus Ziege gegen humanes kardiales Troponin. Der erste Antikörper des Sandwich bindet an das Peptid der Sequenz 2. Er ist mit FITC markiert und wird über anti-FITC auf Magnetpartikeln immobilisiert. Der 2. Antikörper des Sandwich reagiert mit dem anti-Ziege-Antiköper des synthetischen Kalibrators. Er trägt alkalische Phosphatase und katalysiert die Farbreaktion. Die Antikörper-Inkubation erfolgte sequentiell. Auch bei diesem Testverfahren konnte eine der Konzentration der Kalibratorsubstanz proportionale Zunahme der Farbintensität gezeigt werden.

## Patentansprüche

1. Verwendung von einem Konjugat von einem Antikörper mit einem für den Analyten spezifischen Peptid oder Peptidderivat zur Herstellung von Kalibratoren in Immunoassays.

2. Verwendung gemäß Anspruch 1, **dadurch gekennzeichnet, daß** der Antikörper gegen einen anderen im Immunoassay verwendeten Antikörper gerichtet ist.

3. Verwendung gemäß den Ansprüchen 1 und 2, **dadurch gekennzeichnet, daß** es sich bei dem Antikörper um einen monoklonalen oder polyklonalen, oder Fragmente hiervon handelt.

4. Verwendung gemäß den Ansprüchen 1 bis 3, **dadurch gekennzeichnet, daß** die Peptide oder Peptidderivate eine Länge von 4 bis 30 Aminosäuren aufweisen.

5. Verwendung nach Ansprüchen 1 bis 4, **dadurch gekennzeichnet, daß** 1 bis 50 Peptide oder Paptidderivate als Bindestellen in dem Konjugat vorhanden sind.

6. Verwendung nach den Ansprüchen 1 bis 5, **dadurch gekennzeichnet, daß** es sich bei den Peptiden oder Peptidderivaten um Sequenzen aus der Aminosäureabfolge des Troponin I handelt.

7. Verwendung nach den Ansprüchen 1 bis 6, **dadurch gekennzeichnet, daß** es sich bei den Paptiden um die Sequenz 1 und/oder Sequenz 2 handelt.

8. Verwendung des Kalibrators gemäß den Ansprüchen 1 und 7 zur Herstellung von diagnostischen Testmitteln.

## Claims

1. Use of a conjugate of an antibody with a peptide or peptide derivative which is specific for the analyte, for preparing calibrators in immunoassays.

2. Use according to Claim 1, **characterized in that** the antibody is directed against another antibody which is used in the immunoassay.

3. Use according to Claims 1 and 2, **characterized in that** the antibody is a monoclonal or polyclonal antibody, or fragments thereof.

4. Use according to Claims 1 to 3, **characterized in that** the peptides or peptide derivatives have a length of from 4 to 30 amino acids.

5. Use according to Claims 1 to 4, **characterized in that** from 1 to 50 peptides or peptide derivatives are present as binding sites in the conjugate.

6. Use according to Claims I to 5, **characterized in that** the peptides or peptide derivatives are sequences from the amino acid sequence of troponin I.

7. Use according to Claims 1 to 6, **characterized in that** the peptides have the sequence 1 and/or sequence 2.

8. Use of the calibrator according to Claims 1 and 7 for preparing diagnostic testing agents.

## Revendications

1. Utilisation d'un conjugué d'un anticorps avec un peptide ou un dérivé peptidique spécifique de l'analyte pour établir des étalons dans un essais immunologiques.

2. Utilisation suivant la revendication 1, **caractérisée en ce que en ce que** l'anticorps est dirigé contre un autre anticorps utilisé dans l'essai immunologique.

3. Utilisation suivant les revendications 1 et 2, **caractérisée en ce que** l'anticorps est un anticorps monoclonal ou polyclonal ou des fragments de cet anticorps.

4. Utilisation suivant les revendications 1 à 3, **caractérisée en ce que** les peptides ou dérivés peptidiques présentent une longueur de 4 à 30 aminoacides.

5. Utilisation suivant les revendications 1 à 4, **caractérisée en ce que** 1 à 50 peptides ou dérivés peptidiques sont présents comme sites de liaison dans le conjugué.

6. Utilisation suivant les revendications 1 à 5, **caractérisée en ce que** les peptides ou dérivés peptidiques sont des séquences de la succession d'aminoacides de la troponine I.

7. Utilisation suivant les revendications 1 à 6, **caractérisée en ce que** les peptides sont formés de la séquence 1 et/ou de la séquence 2.

8. Utilisation de l'étalon suivant les revendications 1 et 7 pour la réalisation de réactifs diagnostiques.
